# EUROPÄISCHE PATENTSCHRIFT

(11) **EP 1 939 630 B1**
(45) Veröffentlichungstag und Bekanntmachung des Hinweises auf die Patenterteilung: **17.12.2014**
(21) Anmeldenummer: 07021511.6
(22) Anmeldetag: 06.11.2007
(51) Int. Cl.: G01N 35/04, G01N 21/90

(54) **Vorrichtung und Verfahren zum Inspizieren von Behältnissen**
Method and device for inspecting containers
Dispositif et procédé pour l'inspection de récipients

(30) Priorität: 27.12.2006 DE 102006062298
(43) Veröffentlichungstag der Anmeldung: 02.07.2008
(73) Patentinhaber: Krones AG, 93073 Neutraubling (DE)
(72) Erfinder: Niedermeier, Anton, 93326 Offenstetten (DE)
(74) Vertreter: Bittner, Bernhard

(56) Entgegenhaltungen:
- WO-A1-94/08230
- WO-A1-2004/053471
- DE-C1- 10 027 226

## Beschreibung

Die vorliegende Erfindung bezieht sich auf eine Vorrichtung und ein Verfahren zum Inspizieren von Behältnissen und insbesondere von gefüllten und verschlossenen Behältnissen.

Aus dem Stand der Technik sind eine Vielzahl von Vorrichtungen und Verfahren zum Inspizieren von Behältnissen bekannt. So ist es beispielsweise bekannt, die Böden der Behältnisse oder deren Verschlüsse zu kontrollieren. Es besteht jedoch auch ein Bedarf, bereits abgefüllte und verschlossene Behältnisse zu kontrollieren, insbesondere daraufhin, ob sich in dem gefüllten Behältnis Fremdpartikel wie beispielsweise Splitter oder dergleichen befinden.

Aus der WO 94/08230 sind ein Verfahren und eine Vorrichtung zum Inspizieren transparenter Gefäße und ihrer flüssigen Inhalte bekannt. Dabei werden in einem ersten Karussell die Gefäße mit geringer Drehgeschwindigkeit einmal vollständig um ihre Hochachse gedreht und dabei die Seitenwand von einer Kamera zur Erkennung von Beschädigungen betrachtet. Im Anschluss daran wird die Drehgeschwindigkeit erhöht, um die Flüssigkeit in Rotation zu versetzen, und um eventuell vorhandene Fremdkörper vom Boden aufzuwirbeln. Noch vor dem Verlassen des ersten Karussells wird die Drehbewegung der Gefäße angehalten, um diese anschließend über ein erstes Sternrad, eine Teilungsverzugsschnecke und ein zweites Sternrad an ein zweites Karussell zu übergeben, in dem die Gefäße ohne eine Eigendrehung um ihre vertikale Achse von mitlaufenden Kameras zur Erkennung von Fremdkörpern im Füllgut inspiziert werden. Durch den vergleichsweise hohen Abstand zwischen dem ersten Karussell und der Inspektionseinrichtung tritt jedoch dabei das Problem auf, dass insbesondere bei geringen Durchlaufgeschwindigkeiten die ursprünglich bewegte Flüssigkeit wieder zur Ruhe gekommen ist, sich daher die eventuellen Fremdkörper bereits wieder abgesetzt haben und damit nicht mehr zuverlässig beobachtet werden können.

Die WO 2004/053471 A1 beschreibt insofern eine Verbesserung, als die Gefäße aus dem ersten Karussell unmittelbar in ein zweites Karussell, in dem eine Inspektionseinheit angeordnet ist, überführt werden. Auf diese Weise kann die Wegstrecke bis zur Beobachtung der Gefäße und damit auch die dafür benötigte Zeit reduziert werden. Genauer gesagt werden bei dieser Vorrichtung in einem ersten Drehkarussell die Behältnisse in einem ersten Drehabschnitt beschleunigt, in einem zweiten Wegabschnitt wird eine bestimmte Drehung beibehalten und in einem dritten Wegabschnitt wird die Drehung der Gefäße um deren eigene Längsachse wieder verzögert.

Bei einer weiterentwickelten Ausführung dieser Inspektionsvorrichtung werden die Behältnisse durch individuelle elektromotorische Antriebe in Drehung versetzt. Diese Vorrichtung arbeitet bei maximaler Produktionsleistung der Inspektionsvorrichtung mit hoher Erkennungssicherheit. Falls jedoch bei niedriger Produktionsleistung gearbeitet wird, tritt das Problem auf, dass die Rotation der Flüssigkeit beim Erreichen der Inspektionseinheit bereits wieder so niedrig sein kann, dass keine Aufwirbelungen von Fremdkörpern mehr auftreten. Die Drehgeschwindigkeit der Behältnisse bzw. der Flüssigkeit in den Behältnissen ist derzeit durch eine Vielzahl von Faktoren limitiert wie beispielsweise das maximal mögliche Beschleunigungsmoment, die Zeit, die die Behältnisse in dem ersten Drehkarussell verbringen und die maximal aufbringbare Bremskraft. Dabei wird unabhängig von der momentanen Produktionsleistung der Inspektionsvorrichtung mit nur einem Bewegungsprofil gearbeitet, wobei dieses Bewegungsprofil, wie oben erwähnt, für die maximale Produktionsleistung optimiert ist und immer an einer ortsfesten, nahe an der Zuführstelle der Behältnisse in das erste Karussell liegenden Stelle gestartet und an einer dazu beabstandeten ortsfesten Position durch Einleiten der Behälterdrehverzögerung abgeschlossen wird, d.h. die Zeitspanne vom Stopp der Drehbewegung der Behältnisse bis zum Erreichen der nachfolgenden Inspektionseinrichtung verlängert sich wegen der bis zum Erreichen des Stillstands annähernd zeitkonstanten Drehverzögerung mit abnehmender Produktionsleistung.

Der vorliegenden Erfindung liegt daher die Aufgabe zugrunde, eine Vorrichtung und ein Verfahren zum Inspizieren von gefüllten und verschlossenen Behältnissen zur Verfügung zu stellen, welche auch bei niedrigeren Produktionsleistungen und allgemein bei unterschiedlichen Produktionsleistungen eine stets gleich bleibend sichere Inspektion der Behältnisse auf Fremdkörper in der Flüssigkeit erlaubt.

Dies wird erfindungsgemäß durch eine Vorrichtung nach Anspruch 1 und ein Verfahren nach Anspruch 9 erreicht. Vorteilhafte Ausführungsformen und Weiterbildungen sind Gegenstand der Unteransprüche.

Die erfindungsgemäße Vorrichtung zum Inspizieren von gefüllten und geschlossenen Behältnissen weist eine Transporteinrichtung auf, welche die Behältnisse eine vorgegebene Wegstrecke mit einer vorgegebenen Transportgeschwindigkeit transportiert und auf der eine Vielzahl von Halteelementen für die Behältnisse angeordnet ist, wobei diese Halteelemente wenigstens teilweise drehbar sind (bzw. zumindest Bestandteile der Haltelemente drehbar sind), um die Behältnisse in eine Rotation um deren Längsachse zu versetzen. Weiterhin ist eine Steuereinrichtung vorgesehen, die bewirkt, dass die Rotation der Behältnisse um deren Längsachse mit einem vorgegebenen Bewegungsprofil gesteuert wird, wobei die Drehung in einem ersten vorgegebenen Wegabschnitt der Behältnisse auf der Wegstrecke gegenüber der Umgebung bis zu einer vorgegebenen maximalen Drehzahl beschleunigt wird und in einem wenigstens zweiten vorgegebenen Wegabschnitt der Behältnisse auf der Wegstrecke bevorzugt bis zum Stillstand der Drehung verzögert wird. Dazwischen kann sich ein Wegabschnitt mit konstanter Drehzahl befinden.

Erfindungsgemäß ist das vorgegebene Bewegungsprofil in Abhängigkeit von einer momentanen Transportgeschwindigkeit der Behältnisse veränderbar.

Unter einem Halteelement werden solche Elemente verstanden, die das Behältnis an einer vorgegebenen Position anordnen und eine Drehung der Behältnisse ermöglichen. Bei dem Halteelement kann es sich auch um einen Drehteller handeln, auf dem ein Behältnis abgestellt wird. Daneben können die Halteelemente auch insbesondere drehbare Greifelemente aufweisen, um die Behältnisse axial einzuspannen. Diese Greifelemente können beispielsweise am Hals oder Kopf der Behältnisse angreifen.

Bei der vorgegebenen Wegstrecke, welche die Behältnisse transportiert werden, kann es sich um einen Kreissektor handeln, es wäre jedoch auch möglich, dass es sich um geradlinige oder in beliebiger Weise gekrümmte Wegabschnitte handelt.

Liegt beispielsweise als Transporteinrichtung ein Drehkarussell vor, wäre es möglich, dass die Behältnisse unter einem gewissen Drehwinkel jeweils von einem speziellen Haltelement aufgenommen werden und beispielweise die Rotation der Behältnisse in einem Winkelabstand von 30° Grad nach der Aufnahme der Behältnisse eingeleitet wird. Damit wird bei dieser Ausführungsform die Drehbewegung in Abhängigkeit vom Ort des Behältnisses gegenüber der Umgebung begonnen, d.h. das Drehbewegungsprofil ist von der Position der Behältnisse auf der Wegstrecke abhängig.

Dieses Bewegungsprofil ist im Stand der Technik unabhängig von der Transportgeschwindigkeit der Behältnisse. Daher wird im Stand der Technik die Drehbewegung immer am gleichen Punkt begonnen, immer auf eine vorgegebene maximale Drehgeschwindigkeit beschleunigt, mit konstanter Drehzahl in Rotation gehalten und nachfolgend an einem immer gleichen festen Punkt beginnend mit vorgegebener Beschleunigung verzögert. Da im Stand der Technik auch stets dieselbe Verzögerungskonstante verwendet wird, ändert sich in Abhängigkeit von der Transportgeschwindigkeit auch derjenige Ort, an dem das Behältnis hinsichtlich seiner Rotation um die eigene Achse zum Stillstand kommt. Damit ändert sich auch in Abhängigkeit von der Transportgeschwindigkeit der Ort, an dem die Flüssigkeit innerhalb des Behältnisses zum Stillstand kommt und insbesondere bei geringen Transportgeschwindigkeiten wird für die Drehbewegung der Behältnisse um die eigene Achse nicht die vollständige Wegstrecke bis zum Erreichen der eigentlichen Inspektionseinrichtung ausgenutzt.

Gegenüber dem Stand der Technik ist das Bewegungsprofil für die maximale Produktionsleistung optimiert, dass heißt, es wird versucht, in der Zeit, in der die Behältnisse in der Transporteinrichtung beispielsweise in einem Beschleunigungskarussell verbleiben, so viel Energie in das Produkt zu bringen, dass Fremdkörper, wie Scherben, innerhalb der Flüssigkeit sich noch ausreichend bewegen, bis das Behältnis die nachfolgende Inspektionseinrichtung erreicht. Damit wird im Stand der Technik bei niedriger Produktion dieselbe Energie eingebracht, obwohl der Behälter länger als bei hoher Leistung in der Transporteinrichtung ist. Die Energie ist aufgrund des inneren Reibverlusts bei langsamer Produktion aufgebraucht, bevor das Behältnis die Inspektionseinheit erreicht hat. Dieses Problem ist insbesondere bei solchen auch als Full-Bottle-Inspection (FBI) bezeichneten Vorrichtungen relevant, bei denen zwischen der Transporteinrichtung und der nachfolgenden Inspektionseinheit Transfereinheiten, wie Transferschnecken vorhanden sind.

Durch die erfindungsgemäße Veränderung des Bewegungsprofils in Abhängigkeit von einer Transportgeschwindigkeit ist es beispielsweise möglich, bei niedriger Produktion eine höhere Rotationsenergie in die Flüssigkeit im Behältnis einzubringen. Auch wäre es möglich, die Drehung erst an einem späteren Ort wieder zu verzögern, um damit zu erreichen, dass auch zum Zeitpunkt der Inspektion eventuelle Fremdkörper noch aufgewirbelt werden.

Falls sich also das Beschleunigungsprofil beziehungsweise Bewegungsprofil dahingehend ändert, dass in die jeweiligen Behältnissen beim Verweilen in der Transporteinrichtung deutlich mehr Energie eingebracht wird, können auch längere Zeiten überbrückt werden, bis ein nachfolgendes Inspektionskarussell erreicht wird. Auch damit kann die minimale Produktionsleistung herabgesetzt werden und die Einplanung der Anlagen vereinfacht sich hierdurch. Durch die längere Verweilzeit in der Transporteinrichtung können, bei Einhaltung der Restriktionen wie beispielsweise der zulässigen Drehbeschleunigungen, höhere Drehzahlen erreicht werden.

So wirkt sich beispielsweise eine Drehzahlerhöhung um 40 % also zum Beispiel von 600 Umdrehungen pro Minute auf 850 Umdrehungen pro Minute als Verdoppelung des Energieniveaus aus. Theoretisch könnte somit die bisherige minimale Produktionsleistung bei gleicher Inspektionssicherheit deutlich, zum Beispiel auf die Hälfte, herabgesetzt werden.

Die Steuereinrichtung steuert bei einer bevorzugten Ausführungsform die maximale Drehzahl der Behältnisse um deren Längsachse in Abhängigkeit von der Transportgeschwindigkeit der Behältnisse in der Transporteinrichtung. So wäre es möglich, einen Beschleunigungsvorgang über eine längere Zeit hinweg durchzuführen und damit ohne Überschreiten der Limitierung für die maximal mögliche Beschleunigung eine höhere Endgeschwindigkeit der Behältnisse zu erreichen.

Bei einer weiteren vorteilhaften Ausführungsform ist die Lage des zweiten Wegabschnitts bezüglich der Wegstrecke in Abhängigkeit von der Transportgeschwindigkeit der Transporteinrichtung veränderbar. So kann beispielsweise bei niedrigerer Transportgeschwindigkeit der zweite Wegabschnitt weiter in Richtung des Endes der Wegstrecke verschoben werden, um auf diese Weise die Zeitspanne bis zur Inspektion der Behältnisse zu verkürzen. Genauer gesagt ist wenigstens der Anfangspunkt des zweiten Wegabschnitts, d.h. der Punkt, ab dem die Drehung der Behältnisse wieder verzögert wird, veränderbar.

In einer weiteren bevorzugten Ausführungsform ist die Transporteinrichtung ein Transportkarussell. Dies bedeutet, dass eine Vielzahl von Halteelementen derart angeordnet ist, dass die Behältnisse im Wesentlichen entlang einer Kreislinie transportiert werden. In diesem Fall werden die einzelnen Wegabschnitte durch die jeweiligen Kreissektoren dieser Bewegung definiert. Das Transportkarussell wird im Folgenden auch als Beschleunigungskarussell bezeichnet.

In einer weiteren vorteilhaften Ausführungsform bewirkt die Steuereinrichtung, dass der erste Wegabschnitt bezüglich der Wegstrecke in Abhängigkeit von der Transportgeschwindigkeit der Transporteinrichtung veränderbar ist. Auch durch eine derartige Veränderung kann die in die Behältnisse eingebrachte Energie verändert werden, beispielsweise ein in Transportrichtung gesehen späteres Losdrehen der Behälter.

Die vorliegende Erfindung ist weiterhin auf eine Anlage zum Inspizieren von gefüllten und verschlossenen Behältnissen gerichtet, welche eine Vorrichtung der oben beschriebenen Art aufweist und ein stromabwärts bezüglich dieser Vorrichtung vorgesehenes zweites Transportkarussell, an dem wenigstens eine Inspektionseinrichtung zur Erkennung von Fremdkörpern in den Behältnissen vorgesehen ist. Bei dieser Inspektionseinrichtung handelt es sich um die eingangs beschriebene Inspektionseinrichtung, die den Inhalt der Behältnisse auf eventuelle Fremdkörper prüft. Vorzugsweise handelt es sich hierbei um eine Inspektionseinrichtung, die nach einer Dunkelfeldmethode arbeitet. Diese Methode erlaubt mit Hilfe reflektierenden Lichts die Sichtbarmachung feiner Strukturdetails, insbesondere auch lichtstreuender Details wie Glassplitter oder dgl. Fremdstoffe.

Bei einer weiteren bevorzugten Ausführungsform weist das zweite Transportkarussell eine Vielzahl von weiteren Halteelementen zur Aufnahme der Behältnisse auf, wobei die Halteelemente des zweiten Transportkarussells direkt die Behältnisse von den Halteelementen der oben beschriebenen Vorrichtung übernehmen. Auf diese Weise ist eine möglichst zeitsparende Übergabe der Behältnisse von der Transporteinrichtung an das zweite Transportkarussell möglich und damit kann der Zeitverlust bis zur Inspektion der Behältnisse möglichst gering gehalten werden. Es können jedoch auch weitere Transporteinheiten zwischen der Transporteinrichtung und dem zweiten Transportkarussell vorgesehen sein, beispielsweise ein Transfersternrad.

In einer weiteren bevorzugten Ausführungsform greifen die Halteelemente des zweiten Transportkarussells die Behältnisse an einer Behältnisumfangswand. Auf diese Weise ist eine Beobachtung des Behältnisbodens möglich, ohne dass diese Beobachtung durch die Halteelemente des zweiten Transportkarussells gestört wird.

Die vorliegende Erfindung ist weiterhin auf ein Verfahren zum Inspizieren von verschlossenen und gefüllten Behältnissen gerichtet. Dabei werden in einem ersten Verfahrensschritt die Behältnisse an eine erste Transporteinrichtung übergeben. In einem weiteren Verfahrensschritt werden die Behältnisse mit der ersten Transporteinrichtung auf einer vorgegebenen Wegstrecke mit einer vorgegebenen Transportgeschwindigkeit transportiert. Weiterhin werden während des Transports der Behältnisse diese um deren Längsachse gedreht, wobei das Bewegungsprofil der Drehung in vorgegebener weise von dem Ort der Behältnisse auf der Wegstrecke gegenüber der Umgebung abhängt. In einem ersten vorgegebenen Wegabschnitt wird dabei die Drehung der Behältnisse auf der Wegstrecke gegenüber der Umgebung bis zu einer vorgegebenen maximalen Drehzahl beschleunigt und in einem weiteren vorgegebenen Wegabschnitt der Behältnisse auf der wegstrecke verzögert und bevorzugt bis zum Stillstand verzögert. Neben einer Drehung der Behältnisse um deren Längsachse ist jedoch auch eine Drehung der Behältnisse um hiervon leicht abweichende Achsen, dass heißt seitlich versetzte oder schräg angeordnete Achsen möglich. Daneben wäre prinzipiell auch eine andere Art der Bewegung denkbar, um eine Bewegung der Flüssigkeit gegenüber dem Behältnis zu erzeugen, wie beispielsweise Schüttelbewegungen und dergleichen.

Erfindungsgemäß wird das vorgegebene Bewegungsprofil in Abhängigkeit von der Transportgeschwindigkeit der ersten Transporteinrichtung gesteuert. Vorteilhafterweise wird die maximale Drehzahl in Abhängigkeit von der Transportgeschwindigkeit gesteuert. Dabei erfolgt diese Steuerung bevorzugt in einem umgekehrten Zusammenhang, d.h. bei niedrigerer Transportgeschwindigkeit wird die maximale Drehzahl erhöht und umgekehrt bei höherer Transportgeschwindigkeit wird die maximale Drehzahl abgesenkt.

Bei einer weiteren vorteilhaften Variante wird die Lage des zweiten Wegabschnitts gegenüber der Umgebung in Abhängigkeit von der Transportgeschwindigkeit der ersten Transporteinrichtung gesteuert.

In einer weiteren vorteilhaften Variante wird die Drehzahl der Behältnisse ausgehend von dem ersten Wegabschnitt mit einer vorgegebenen Beschleunigung bis zu einer Maximaldrehzahl erhöht und die Beschleunigung unter Berücksichtigung der Transportgeschwindigkeit der ersten Transporteinrichtung gesteuert. Dabei kann insbesondere eine Nebenposition definiert werden, bei der ein Beschleunigungsvorgang für die Drehung der Behältnisse abgeschlossen ist. Auch kann eine Nebenposition definiert werden, bei der eine Verzögerung der Drehung der Behältnisse eingeleitet wird. Auch die Lage dieser Nebenpositionen kann dabei in Abhängigkeit von der Transportgeschwindigkeit der ersten Transporteinrichtung variabel sein.

Vorzugsweise wird die Drehzahl der Behältnisse zwischen dem ersten Wegabschnitt und dem zweiten Wegabschnitt im Wesentlichen konstant gehalten. Auf diese Weise kann eine besonders gleichmäßige Beschleunigung der Flüssigkeit in den Behältnissen erreicht werden.

Weitere Vorteile und Ausführungsformen ergeben sich aus den beigefügten Zeichnungen:

Darin zeigen:
- Fig. 1: eine erfindungsgemäße Anlage zum Inspizieren von Behältnissen;
- Fig. 2: ein konstantes Bewegungsprofil nach dem Stand der Technik;
- Fig. 3: eine Veranschaulichung der erfindungsgemäßen Änderung des Bewegungsprofils; und
- Fig. 4: eine weitere Variante der erfindungsgemäßen Änderung des Bewegungsprofils.

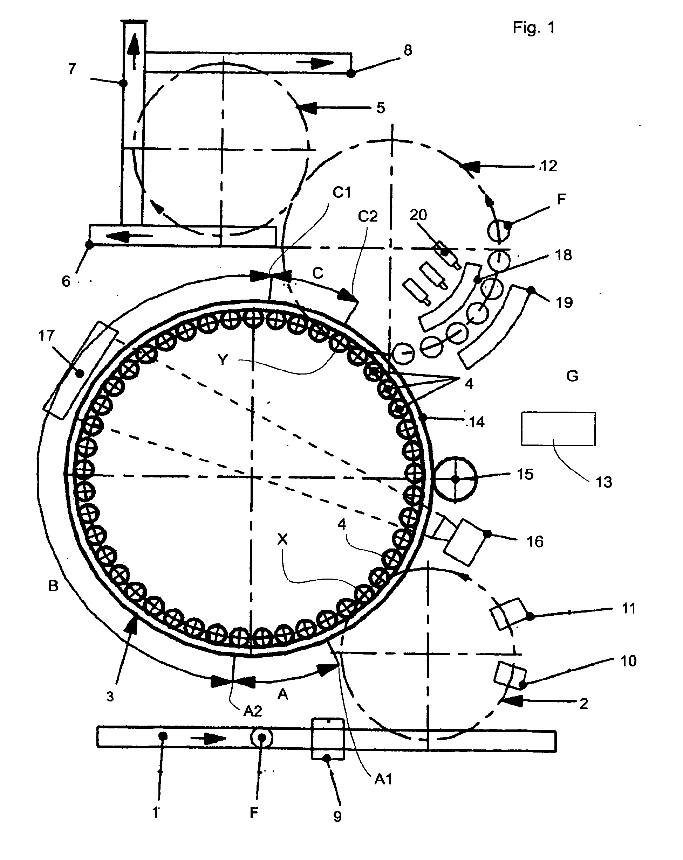

Die in Fig. 1 schematisch dargestellte Vorrichtung zum Inspizieren von gefüllten und verschlossenen Behältnissen (im vorliegenden Fall handelt es sich um Getränkeflaschen) ist speziell zum Inspizieren von Flaschen aus transparenten oder semitransparenten Material eingerichtet. Die zu überprüfenden Flaschen F werden beispielsweise auf einer vorgeordneten Flaschenfüll- und Verschließmaschine über ein Einlaufförderband 1 vorzugsweise kontinuierlich einem Einlaufsternrad 2 zugeführt.

Dabei passieren die Flaschen vor der Übernahme durch das Einlaufsternrad 2 eine stationär angeordnete Einlaufkontrolle 9, die das Vorhandensein eines Verschlusses und gegebenenfalls auch die Füllhöhe überprüft. Zur Vermeidung von zu starken Beschleunigungen der Behältnisse, welche wiederum zu Verschmutzungen der Inspektionseinheiten durch überschwappende Flüssigkeit aus unverschlossenen Flaschen führen können, werden diese fehlerhaft verschlossenen Flaschen F vom Einlaufsternrad 2 nicht erfasst und passieren es lediglich in tangentialer Richtung und in Richtung einer nachgeordneten Sammelstelle. In gleicher Weise wird auch mit über- oder unterfüllten Flaschen verfahren.

Das Einlaufsternrad 2 wird hier gegen den Uhrzeigersinn kontinuierlich angetrieben und weist eine Vielzahl von bevorzugt in gleichmäßigem Maschinenteilungsabstand angeordneten, selektiv ansteuerbaren Greifelementen zum Erfassen der Flaschen am Rumpf und gegebenenfalls auch Kopf- bzw. Halsbereich. Diese Greifelemente sind selektiv ansteuerbar und an unterschiedliche Flaschendurchmesser anpassbar.

Während des Transfers vom Einlaufförderband 1 zu einer in Umlaufrichtung folgenden Transporteinrichtung 3 werden die Flaschen bodenfrei zunächst über eine stationäre Bodenabblaseinrichtung 10 zum Entfernen von Seifenschaum oder dergleichen sowie eine danach angeordnete, im Hellfeldverfahren betreibbare Bodenkontrollstation 11 zum Erkennen von Verschmutzungen oder Schäden am Flaschenboden selbst hinwegbewegt. Diese Bodenkontrollstation 11 dient auch zur Erkennung von schweren, durch Drehen der Flaschen nicht aufwirbelbaren Fremdkörpern.

Die konkrete Ausgestaltung des Einlaufsternrads 2 ist aus dem Stand der Technik bekannt und wird daher nicht genauer erläutert. So wird beispielsweise auf die europäischen Patente 0 726 216 B1 und 0 743 267 B1 für das Einlaufsternrad 2 und das deutsche Gebrauchsmuster 94 01 926 U1 für die Bodenabblaseinrichtung und die deutsche Patentanmeldung 101 33 104 für die Bodenkontrollstation 11 hingewiesen. Der Offenbarungsgehalt dieser Druckschriften wird ausdrücklich mit einbezogen. Ebenso wird der Offenbarungsgehalt der eingangs erwähnten WO 2004/053471 ausdrücklich durch Bezugnahme zum Gegenstand dieser Offenbarung gemacht.

Im Berührpunkt X der Teilkreise des Einlaufsternrads 2 und der im Uhrzeigersinn umlaufenden Transporteinrichtung 3 werden die Flaschen F mit ihrer Bodenfläche auf um eine vertikale Achse in der Transporteinrichtung 3 drehbar gelagerte Drehteller 4 übergeben und in einer axialen Einspannung drehbar gehalten. Dies ist ebenfalls aus dem Stand der Technik bekannt.

Im weiteren Verlauf werden die auf den Drehtellern 4 aufrecht stehenden Flaschen zunächst beim Passieren des ersten Umlaufsektors beziehungsweise Wegabschnitts A der Transporteinrichtung 3 um ihre Längsachse kontinuierlich beschleunigt in Drehung versetzt, um anschließend den Wegabschnitt B mit einer definierten maximalen Drehzahl zu durchfahren. An diesen Wegabschnitt B schließt sich ein Umlaufsektor beziehungsweise Wegabschnitt C an, in welchem die Flaschendrehung bevorzugt kontinuierlich annähernd bis zum Stillstand abgebremst wird.

Bei der in Fig. 1 gezeigten Ausführungsform weist jeder Drehteller 4 ein unten liegendes (nicht gezeigtes) auf einer Welle drehbar gelagertes Ritzel auf, dass mit der Innenverzahnung eines eine Innen- und Außenverzahnung aufweisenden Zahnkranzes 14 kämmt, wobei sich dieser Zahnkranz über eine mittenfreie Kugeldrehverbindung auf der Gestellplatte G der Maschine abstützt. Dieser Zahnkranz 14 ist durch ein in seine Außenverzahnung eingreifendes Antriebszahnrad 15 gesteuert von einem drehzahlveränderbaren Antrieb antreibbar. Bei diesem Antrieb kann es sich um einen Elektromotor oder dergleichen handeln. Der Antrieb erfolgt dem Uhrzeigersinn entgegengerichtet und damit gegensinnig zum Karussell bzw. der Transporteinrichtung 3.

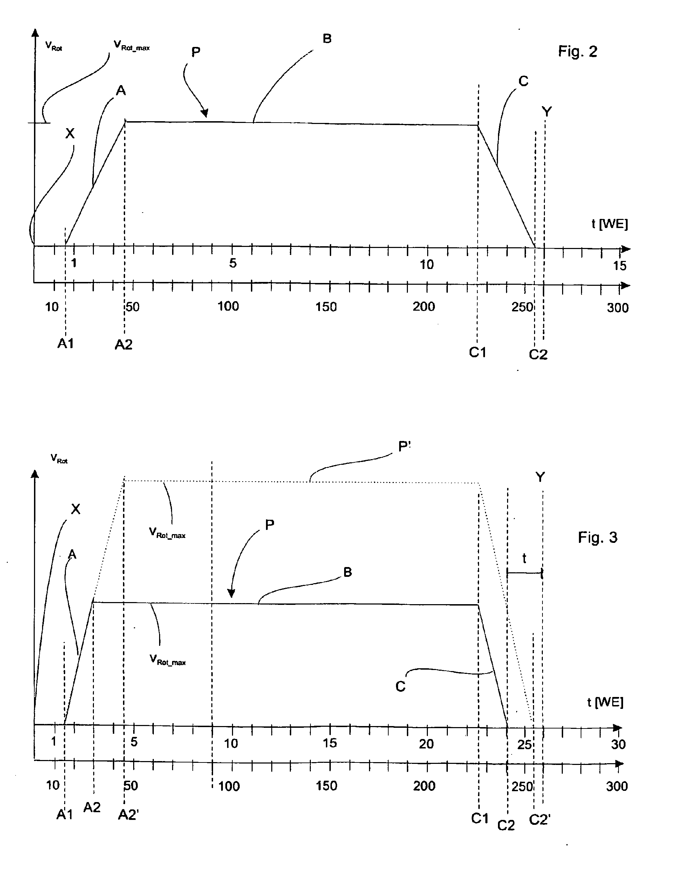

Dieser Gegenlauf ermöglicht eine ausreichend hohe Rotation der Drehteller 4. Es wäre jedoch in einer bevorzugten Ausführungsform auch möglich, die einzelnen Drehteller 4 jeweils durch separate Antriebseinrichtungen wie Elektromotoren separat anzutreiben, um auf diese Weise eine individuelle Steuerung der einzelnen Rotationen der Flaschen zu erreichen. Auch wäre es möglich, mehrere Drehteller 4, beispielsweise die Drehteller 4 in einem bestimmten Segment der Transporteinrichtung 3 jeweils gemeinsam zu steuern. In der in Fig. 2 gezeigten Ausführungsform ist es ferner möglich, für jeden Drehteller 4 steuerbare Magnetkupplungen vorzusehen, die auf die Drehteller 4 beim Durchfahren der oben genannten Umlaufsektoren bzw. Abschnitte A, B, C das übertragende Drehmoment betreffend mehr oder weniger stark übertragen und so eine individuelle Steuerung der Drehbewegung der einzelnen Drehteller ermöglichen. Diese Magnetkupplung entspricht der in der WO 2004/0534 71 beschriebenen Magnetkupplungen und wird daher nicht eingehend beschrieben.

Das Bezugszeichen 17 bezieht sich auf einen Seitenwandleuchtschirm, der an der äußeren Seite der Transporteinrichtung 3 angeordnet ist. Daneben ist eine Seitenwandkamera 16 vorgesehen, die diesem Seitenwandleuchtschirm 17 diametral gegenüberliegt. Auf diese Weise wird eine Hellfeldinspektion im Durchlicht ermöglicht, wodurch beispielsweise Abdunkelungen, die durch Beschädigungen bzw. Verschmutzungen erzeugt werden, erkannt werden können.

Den Endbereich des Wegabschnitts C der Transporteinrichtung 3 tangiert ein zweites Transportkarussell 12, das - in gleicher Weise wie das Einlaufsternrad 2 - an seiner Peripherie eine Vielzahl von mit dem Maschinenteilungsabstand versetzt angeordnete, selektiv steuerbare Greifelemente zum Erfassen der Flaschen an ihrem Rumpf und/oder gegebenenfalls auch am Kopf- bzw. Halsbereich aufweist.

Auf diese Weise können die Flaschen F im gemeinsamen Berührpunkt Y zu der ersten Transporteinrichtung 3 erfasst und bodenfrei gegen den Uhrzeigersinn in Richtung eines darauffolgenden Sortiersternrads 5 transferiert werden. Auf dem Weg dorthin werden die Flaschen F einer Fremdkörpererkennung im Dunkelfeldverfahren unterzogen, mit der lichtstreuende Fremdkörper, insbesondere transparente Glassplitter, erkennbar sind.

Zu diesem Zweck sind beiderseits der gekrümmten Umlaufbahn der Flaschen F an die Bahnkrümmung angepasste, äquidistante Leuchtschirme 18 und 19 stationär angeordnet, zwischen denen die Flaschen F frei durchlaufen und dabei seitlich möglichst großflächig beleuchtet werden können.

Durch die gleichzeitig erfolgende beidseitige, tunnelartige Beleuchtung ist eine sehr hohe Lichteinleitung in die Flaschen erreichbar, die insbesondere bei trüben oder dunklen Flüssigkeiten, wie beispielsweise hefehaltigem Bier oder Cola von Vorteil ist.

Die Leuchtschirme 18 und 19 sind bevorzugt mit einer Vielzahl von LEDs bestückt, die durch eine Beleuchtungssteuerung gepulst betreibbar sind.

Daneben ist das zweite Transportkarussell 12 mit unterhalb seiner nicht dargestellten Greifelemente angeordneten Kameras 20 bestückt, wobei beispielsweise jeweils eine Kamera pro Greifelement vorgesehen ist, die gemeinsam mit den Greifelementen stellungssynchron umlaufen und den Boden der beleuchteten Flaschen beobachten. Mit dieser Anordnung wird eine Dunkelfeldbeleuchtung realisiert, in der lichtstreuende Fehler bzw. Fremdkörper in einer ansonsten dunklen Abbildung als helle Punkte oder Zonen aufscheinen.

Auch können durch diese Vorgehensweise Fremdkörper in der Flüssigkeit unterschieden werden von Fremdkörpern bzw. Fehlstellen in dem Gefäß selbst. Fremdkörper oder Beschädigungen in dem Gefäß selbst bleiben während der Bewegung der Flaschen in dem zweiten Karussell 12 im Wesentlichen stationär. Fremdkörper in der Flüssigkeit hingegen werden sich, sofern sich die Flüssigkeit noch gegenüber den Flaschen bewegt, ihren Ort zeitabhängig ändern.

Alternativ wäre auch eine stationäre Anordnung einer oder mehrerer Kameras denkbar, wobei durch eine gleichzeitige Ansteuerung der Kameras 20 zur Bildaufnahme sowie der LEDs der Leuchtschirme 18 und 19 vorteilhafterweise eine sonst erforderliche Triggerung entfallen kann.

Das Bezugszeichen 5 bezieht sich auf ein Sortiersternrad, das ebenfalls eine Vielzahl von selektiv steuerbaren, nicht dargestellten Greifelementen aufweist. Durch dieses Sortiersternrad können die inspizierten Flaschen in Abhängigkeit der Prüfergebnisse der Bodenkontrollstation 11, der Seitenwandkamera 16 und der die Flaschen F durch den Boden betrachtenden Kameras 20 auf verschiedene Förderbänder abgegeben werden. So können beispielsweise die unbeanstandeten Flaschen die Inspektionsmaschine über das mit 6 bezeichnete Auslaufförderband verlassen, während Fehler aufweisende Flaschen in Abhängigkeit des erkannten Fehlers wahlweise auf die Ausschleusbänder 7 oder 8 ausleitbar sind.

Wie eingangs erwähnt, ist jedoch Voraussetzung für eine effektive Feststellung des Vorhandenseins von Fremdkörpern in der Flüssigkeit, dass sich die Flüssigkeit auch gegenüber dem Behältnis bewegt bzw. diese Fremdkörper noch aufgewirbelt werden, wenn eine Beobachtung durch die Kameras 20 erfolgt.

Das Bezugszeichen 13 bezieht sich auf eine Steuereinrichtung, die die Bewegungsprofile der Greifelemente bzw. die Drehung der Flaschen um deren Längsachse steuert.

Fig. 2 zeigt einen Verlauf eines ortsfesten und konstanten Bewegungsprofils (bei max. Leistung) nach dem Stand der Technik.

Dabei korrespondieren die jeweiligen Abschnitte A, B und C zu den entsprechenden in Figur 1 gezeigten Wegabschnitten A, B, C und auf der unteren X-Achse wurde eine Einteilung in Grad angegeben, wobei die Position 0° die Position X der Übergabe der Flaschen von dem Einlaufsternrad 2 an die Transporteinrichtung 3 anzeigt und die Position 260° die Position Y der Übergabe der Flaschen von der Transporteinrichtung 3 an das zweite Karussell 12.

In dem Wegabschnitt A werden die Flaschen um ihre Längsrichtung gedreht und dabei, wie aus Figur 2 ersichtlich, beschleunigt bis zu einer Maximaldrehzahl V_{rot_Max}. Genauer gesagt wird die Drehung der Flaschen an dem ortsfesten durch die Linie A1 kennzeichneten Ort begonnen und bis zu dem Ort A2 mit einer konstanten Beschleunigung beschleunigt. Der Ort A1 liegt damit, unabhängig von der Transportgeschwindigkeit V_{Trans}, bei ca 15° (vgl. die untere X- Achse).

In dem Abschnitt B, der sich hier über ca. 180° erstreckt, wird die Drehung der Flaschen im Wesentlichen konstant gehalten, wie ebenfalls aus Figur 2 ersichtlich. Schließlich wird in dem Bewegungsabschnitt C die Drehung der Flaschen wieder verzögert. Die vertikale Linie bei C2 deutet innen die Position an, an der die Flaschendrehung wieder im Wesentlichen angehalten hat. Kurz nach dieser Position wird die Flasche, wie oben erwähnt, an das zweite Transportkarussell 12 übergeben. Im Stand der Technik ist der in Figur 2 gezeigte Bewegungsverlauf P konstant und von der Transportgeschwindigkeit unabhängig.

Dies bedeutet, dass die Bewegung nur vom jeweiligen Ort der Flaschen abhängt. Die obere X-Achse bezieht sich auf eine Zeiteinteilung mit willkürlichen Zeiteinheiten. In dieser Transportgeschwindigkeit V_{Trans} der Transporteinrichtung 3 legen die Behältnisse in fünf Zeiteinheiten einen Umfangswinkel von 100° Grad zurück.

Figur 3 zeigt eine andere Arbeitssituation, wobei hier die Transportgeschwindigkeit Vₜᵣₐₙₛ der Transporteinrichtung 3 um die Hälfte reduziert ist. Dies wird insbesondere durch die obere X-Achse erkennbar, bei der hier die doppelte Gesamtzeit von 30 Zeiteinheiten für die vollständige Bewegung benötigt wird. Jedoch wird auch hier bei der gleichen Winkelstellung (Ort A1) die Beschleunigung der Behältnisse gestartet, und ebenfalls wird die Verzögerung der Drehbewegung an der durch C1 bezeichneten Position verzögert.

Da jedoch, wie oben erwähnt, im Stand der Technik die Beschleunigung der Drehgeschwindigkeit V_{Rot} stets konstant gehalten wird und beispielsweise der maximal möglichen Beschleunigung entspricht, wird hier die maximale Drehzahl bei einer früheren Winkelposition erreicht, als bei voller Transportgeschwindigkeit. Dies äußert sich in Figur 3 durch den steileren Anstieg des Bewegungsprofils P in dem Wegabschnitt A. Die Beschleunigung ist, wie üblich, als Geschwindigkeitsänderung pro Zeiteinheit definiert. Damit ist jedoch im Stand der Technik das Bewegungsprofil unabhängig von der Transportgeschwindigkeit V_{Trans}, da die jeweiligen Punkte A1 und C1, an denen die Beschleunigung beziehungsweise die Verzögerung der Drehbewegung begonnen wird, jeweils fest sind. Damit wird, wie sich aus der Bezugnahme auf Figur 3 ergibt, unter einem vom Ort der Flaschen abhängigen Bewegungsprofil verstanden, dass jeweils diejenigen Punkte auf dem Wegabschnitt, an denen die Drehbewegung der Flaschen beschleunigt beziehungsweise verzögert wird, fest sind.

Man erkennt, dass zwischen dem Stillstand der Behältnisse (Position C2) und der Position Y eine gewisse Zeitspanne Δt liegt. Diese Zeitspanne ist bedingt durch die geringere Transportgeschwindigkeit Vₜᵣₐₙₛ und kann im Extremfall dazu führen, dass die Flüssigkeit innerhalb der Behältnisse in dem Moment, in dem die Behältnisse die Inspektionseinrichtungen 20 bzw. die Kameras passieren, bereits zum Stillstand gekommen ist. Dies gilt insbesondere dann, wenn die Transportgeschwindigkeit V_{Trans} der Transporteinrichtung 3 noch weiter verzögert wird.

Daher wird erfindungsgemäß das Bewegungsprofil B unter Berücksichtigung der Transportgeschwindigkeit der Transporteinrichtung 3 verändert. Da die Beschleunigungskraft und die Bremskraft limitiert sind, ist es, wie durch das Bezugszeichen P' angedeutet, möglich, die maximale Drehzahl V_{Rot_max} zu erhöhen. Dies ist in Figur 3 am Beispiel einer Verdoppelung der maximalen Drehzahl V_{Rot_max} dargestellt. In diesem Fall wird durch die Verdoppelung ebenfalls erreicht, dass die Behältnisse in dem Moment der Übergabe gerade zur Ruhe kommen (Position C2'). Gleichfalls wird jedoch sichergestellt, dass die Flüssigkeit in den Behältnissen noch in Bewegung ist, wenn die Flaschen die Inspektionseinrichtung 20 passieren.

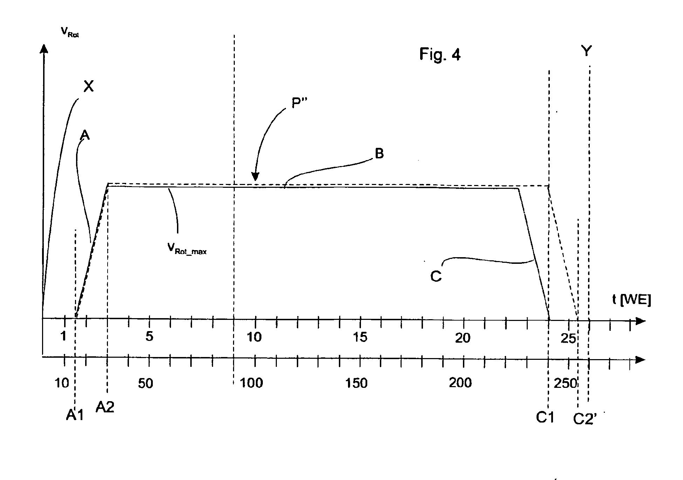

In Figur 4 ist eine weitere Möglichkeit einer derartigen Veränderung des Bewegungsprofils gezeigt. Hier wurde der Verzögerungs- bzw. Bremsvorgang um einen gewissen Zeitversatz Δt verschoben, um auch hier zu erreichen, dass die Flaschen erst in dem Moment der Übergabe an das zweite Karussell 12 bzw. kurz vor diesem Zeitpunkt zur Ruhe kommen (Position C2'). Das entsprechende Bewegungsprofil ist mit dem Bezugszeichen P" gekennzeichnet. Zur Veranschaulichung wurde das Bewegungsprofil, welches an sich teilweise genau über dem Bewegungsprofil P liegt, geringfügig verschoben. Es sind jedoch auch Kombinationen der beiden Bewegungsprofile P' und P'' - je nach Anwendungsfall möglich. So kann einerseits die maximale Drehgeschwindigkeit v_{Rot_max} geringfügig erhöht werden und andererseits auch der Zeitpunkt an dem die Verzögerung eingeleitet wird, geringfügig verschoben werden.

Damit wird sowohl bei dem in Fig. 3 als auch bei dem in Fig. 4 gezeigten Verfahren die Position C2 näher an die Position Y verschoben oder, mit anderen Worten, die Drehung der Flaschen um ihre Längsachse an einer Position C2' angehalten, die näher an der Position Y liegt, als dies im Stand der Technik der Fall ist.

Sämtliche der in den Anmeldungsunterlagen beanspruchten Merkmale werden als erfindungswesentlich beansprucht, sofern sie einzeln oder in Kombination neu und erfinderisch gegenüber dem Stand der Technik sind.

## Patentansprüche

1. Vorrichtung zum Inspizieren von gefüllten und verschlossenen Behältnissen (F) mit einer Transporteinrichtung (3), welche die Behältnisse (F) eine vorgegebene Wegstrecke mit einer Transportgeschwindigkeit vₜᵣₐₙₛ transportiert und an der eine Vielzahl von Halteelementen für die Behältnisse (F) angeordnet ist, wobei diese Halteelemente jeweils drehbar sind, um die Behältnisse (F) in eine Rotation um deren Längsachse zu versetzen, mit einer Steuereinrichtung (13), die bewirkt, dass die Rotation der Behältnisse um deren Längsachse mit einem vorgegebenen vom Ort der Behältnisse auf der Wegstrecke abhängigen Bewegungsprofil (P) gesteuert wird, wobei die Drehung in einem ersten vorgegebenen Wegabschnitt (A) der Behältnisse auf der Wegstrecke bis zu einer vorgegebenen maximalen Drehzahl v_{Rot_max} beschleunigt wird und in einem zweiten vorgegebenen Wegabschnitt (C) der Behältnisse auf der Wegstrecke verzögert wird, **dadurch gekennzeichnet, dass** das vorgegebene Bewegungsprofil (P) in Abhängigkeit von der Transportgeschwindigkeit v_{Trans} der Behältnisse (F) veränderbar ist.

2. Vorrichtung nach Anspruch 1, **dadurch gekennzeichnet, dass** die Steuereinrichtung (13) die maximale Drehzahl v_{Rot_max} der Behältnisse um deren Längsachse in Abhängigkeit von der Transportgeschwindigkeit v_{Trans} der Behältnisse in der Transporteinrichtung (3) steuert.

3. Vorrichtung nach wenigstens einem der vorangegangenen Ansprüche, **dadurch gekennzeichnet, dass** wenigstens die Lage des zweiten Wegabschnitts (C) bezüglich der Wegstrecke in Abhängigkeit von der Transportgeschwindigkeit v_{Trans} der Transporteinrichtung veränderbar ist.

4. Vorrichtung nach Anspruch 1, **dadurch gekennzeichnet, dass** die Transporteinrichtung (3) ein Transportkarussell (3) ist.

5. Vorrichtung nach wenigstens einem der vorangegangenen Ansprüche, **dadurch gekennzeichnet, dass** die Steuereinrichtung bewirkt, dass der erste Wegabschnitt (A) bezüglich der Wegstrecke in Abhängigkeit von der Transportgeschwindigkeit v_{Trans} der Transporteinrichtung (3) veränderbar ist.

6. Anlage (10) zum Inspizieren von gefüllten und verschlossenen Behältnissen mit einer Vorrichtung nach wenigstens einem der vorangegangenen Ansprüche und einem stromabwärts bezüglich der Vorrichtung vorgesehenen zweiten Transportkarussell (12), an dem wenigstens eine Inspektionseinrichtung (18, 19, 20) zur Erkennung von Fremdkörpern in dem Behältnis vorgesehen ist.

7. Anlage nach Anspruch 4, **dadurch gekennzeichnet, dass** das zweite Transportkarussell (12) eine Vielzahl von weiteren Halteelementen zur Aufnahme der Behältnisse (F) aufweist, wobei die Halteelemente des Transportkarussells direkt die Behältnisse von den Halteelementen der Vorrichtung übernehmen.

8. Anlage nach Anspruch 5, **dadurch gekennzeichnet, dass** die Halteelemente des Transportkarussells (12) die Behältnisse (F) an einer Behältnisumfangswand greifen.

9. Verfahren zum Inspizieren von verschlossenen und gefüllten Behältnissen mit den Schritten:
- Übergabe der Behältnisse an eine erste Transporteinrichtung (3)
- Transport der Behältnisse mit der ersten Transporteinrichtung (3) auf einer Wegstrecke mit einer vorgegebenen Transportgeschwindigkeit v_{Trans};
- Drehen der Behältnisse mit einem vorgegebenen vom Ort der Behältnisse auf der Wegstrecke abhängigen Bewegungsprofil (P) um deren Längsachse, wobei die Drehung in einem ersten vorgegebenen Wegabschnitt (A) der Behältnisse auf der Wegstrecke gegenüber der Umgebung bis zu einer vorgegebenen maximalen Drehzahl beschleunigt wird und in einem zweiten vorgegebenen Wegabschnitt (C) der Behältnisse auf der Wegstrecke verzögert wird, **dadurch gekennzeichnet, dass** das vorgegebene Bewegungsprofil (P) in Abhängigkeit von der Transportgeschwindigkeit v_{Trans} der ersten Transporteinrichtung (3) gesteuert wird.

10. Verfahren nach Anspruch 9, **dadurch gekennzeichnet, dass** die maximale Drehzahl v_{Rot_max} in Abhängigkeit von der Transportgeschwindigkeit v_{Trans} gesteuert wird.

11. Verfahren nach wenigstens einem der vorangegangenen Ansprüche 9 - 10, **dadurch gekennzeichnet**, die relative Lage des zweiten Wegabschnitts (C) gegenüber der Umgebung in Abhängigkeit von der Transportgeschwindigkeit v_{Trans} der ersten Transporteinrichtung (3) gesteuert wird.

12. Verfahren nach Anspruch 7, **dadurch gekennzeichnet**, die Drehzahl der Behältnisse ausgehend von dem ersten Wegabschnitt (A) mit einer vorgegebenen Beschleunigung bis zu einer Maximaldrehzahl v_{Rot_max} erhöht wird und die Beschleunigung unter Berücksichtigung der Transportgeschwindigkeit v_{Trans} der ersten Transporteinrichtung (3) gesteuert wird.

13. Verfahren nach wenigstens einem der vorangegangenen Ansprüche 7 - 8, **dadurch gekennzeichnet**, die Drehzahl zwischen dem ersten Wegabschnitt (A) und dem zweiten Wegabschnitt (C) im Wesentlichen konstant gehalten wird.

## Claims

1. Apparatus for inspecting filled and closed containers (F), comprising a transporting device (3) which transports the containers (F) for a preset distance of travel at a speed of transport V_{Trans} and at which are arranged a plurality of holding elements for the containers (F), the holding elements each being rotatable to set the containers (F) rotating about their longitudinal axes, a control device (13) which causes the rotation of the containers about their longitudinal axes to be controlled to follow a preset profile of movement (P) which depends on the position of the containers on the distance of travel, the rotation being accelerated to a preset maximum speed of rotation V_{Rot_max} in a first preset part (A) of the travel of the containers over the distance of travel and being decelerated in a second preset part (C) of the travel of the containers over the distance of travel, **characterized in that** the preset profile of movement (P) is variable as a function of the speed of transport V_{Trans} of the containers (F).

2. Apparatus according to claim 1, **characterized in that** the control device (13) controls the maximum speed of rotation V_{Rot-max} of the containers about their longitudinal axes as a function of the speed of transport V_{Trans} of the containers in the transporting device (3).

3. Apparatus according to at least one of the preceding claims, **characterized in that** at least the position of the second preset part (C) of the travel relative to the distance of travel can be varied as a function of the speed of transport V_{Trans} of the transporting device.

4. Apparatus according to claim 1, **characterized in that** the transporting device (3) is a transporting carousel (3).

5. Apparatus according to at least one of the preceding claims, **characterized in that** the control device causes the first part (A) of the travel to be variable relative to the distance of travel as a function of the speed of transport V_{Trans} of the transporting device (3).

6. Arrangement (10) for inspecting filled and closed containers, having an apparatus according to at least one of the preceding claims and having a second transporting carousel (12) which is provided downstream of this apparatus and at which at least one inspecting device (18, 19, 20) for detecting foreign bodies in the container is provided.

7. Arrangement according to claim 6, **characterized in that** the second transporting carousel (12) has a plurality of further holding elements to receive the containers (F), the holding elements of the transporting carousel taking over the containers directly from the holding elements of the device.

8. Arrangement according to claim 7, **characterized in that** the holding elements of the transporting carousel (12) take hold of the containers (F) by a circumferential wall of the containers.

9. Method of inspecting closed and filled containers, comprising the following steps:
handover of the containers to a first transporting device (3);
transport of the containers by the first transporting device (3) over a distance of travel at a preset speed of transport V_{Trans}; and
rotation of the containers about their longitudinal axes following a profile of movement (P) which depends on the position of the containers along the distance of travel, the rotation being accelerated to a preset maximum speed of rotation in a first preset part (A) of the travel of the containers along the distance of travel relative to the surroundings, and being decelerated in a second preset part (C) of the travel of the containers along the distance of travel, **characterized in that** the preset profile of movement (P) is controlled as a function of the speed of transport V_{Trans} of the first transporting device (3).

10. Method according to claim 9, **characterized in that** the maximum speed of rotation V_{Rot-max} is controlled as a function of the speed of transport V_{Trans}.

11. Method according to at least one of the preceding claims 9 - 10, **characterized in that** the relative position of the second part (C) of the travel with respect to the surroundings is controlled as a function of the speed of transport V_{Trans} of the first transporting device (3).

12. Method according to claim 9, **characterized in that** the speed of rotation of the containers to a maximum speed of rotation V_{Rot-max} at a preset acceleration starting from the first part (A) of the travel is increased and the acceleration is controlled by taking account of the speed of transport V_{Trans} of the first transporting device (3).

13. Method according to at least one of the preceding claims 9 - 10, **characterized in that** the speed of rotation is kept substantially constant between the first part (A) of the travel and the second part (C) of the travel.

## Revendications

1. Système pour l'inspection de récipients (F) remplis et bouchés avec un dispositif de transport (3) qui convoie les récipients (F) sur un chemin défini avec une vitesse de transport Vₜᵣₐₙₛ et sur lequel est disposée une pluralité d'éléments de maintien pour les récipients (F), lesdits éléments de maintien étant respectivement rotatifs pour entraîner en rotation les récipients (F) autour de leur axe longitudinal, avec un dispositif de commande (13) entraînant la commande de rotation des récipients autour de leur axe longitudinal avec un profil de déplacement (P) défini en fonction de la localisation des récipients sur le chemin, la rotation étant accélérée sur un premier segment de chemin (A) défini des récipients jusqu'à une vitesse de rotation maximale V_{Rot_max} définie, et ralentie sur un deuxième segment de chemin (C) défini des récipients, **caractérisé en ce que** le profil de déplacement (P) défini peut être modifié en fonction de la vitesse de transport V_{Trans} des récipients (F).

2. Système selon la revendication 1, **caractérisé en ce que** le dispositif de commande (13) commande la vitesse de rotation maximale V_{Rot_max} des récipients autour de leur axe longitudinal en fonction de la vitesse de transport V_{Trans} des récipients dans le dispositif de transport (3).

3. Système selon au moins une des revendications précédentes, **caractérisé en ce qu'**au moins la position du deuxième segment de chemin (C) par rapport au chemin peut être modifiée en fonction de la vitesse de transport V_{Trans} du dispositif de transport.

4. Système selon la revendication 1, **caractérisé en ce que** le dispositif de transport (3) est un carrousel de transport (3).

5. Système selon au moins une des revendications précédentes, **caractérisé en ce que** le dispositif de commande peut effectuer la modification du premier segment de chemin (A) par rapport au chemin en fonction de la vitesse de transport V_{Trans} du dispositif de transport (3).

6. Installation (10) pour l'inspection de récipients remplis et bouchés avec un système selon au moins une des revendications précédentes et un deuxième carrousel de transport (12) prévu en aval par rapport au système, sur lequel est prévu au moins un dispositif d'inspection (18, 19, 20) pour la détection de corps étrangers dans le récipient.

7. Installation selon la revendication 4, **caractérisé en ce que** le deuxième carrousel de transport (12) comporte une pluralité d'autres éléments de maintien pour la réception des récipients (F), les éléments de maintien du carrousel de transport recevant directement les récipients des éléments de maintien du système.

8. Installation selon la revendication 5, **caractérisé en ce que** les éléments de maintien du carrousel de transport (12) saisissent les récipients (F) sur une paroi périphérique de récipient.

9. Procédé d'inspection de récipients remplis et bouchés, comprenant les étapes suivantes :
- transfert des récipients à un premier dispositif de transport (3)
- transport des récipients par le premier dispositif de transport (3) sur un chemin avec une vitesse de transport V_{Trans} définie ;
- rotation des récipients avec un profil de déplacement (P) autour de leur axe longitudinal défini en fonction de la localisation des récipients sur le chemin, la rotation étant accélérée sur un premier segment de chemin (A) défini des récipients par rapport à l'environnement jusqu'à une vitesse de rotation maximale définie, et ralentie sur un deuxième segment de chemin (C) défini des récipients, **caractérisé en ce que** le profil de déplacement (P) défini est commandé en fonction de la vitesse de transport V_{Trans} du premier dispositif de transport (3).

10. Procédé selon la revendication 9, **caractérisé en ce que** la vitesse de rotation maximale V_{Rot_max} est commandée en fonction de la vitesse de transport V_{Trans}.

11. Procédé selon au moins une des revendications 9 et 10, **caractérisé en ce que** la position relative du deuxième segment de chemin (C) par rapport à l'environnement est commandée en fonction de la vitesse de transport V_{Trans} du premier dispositif de transport (3).

12. Procédé selon la revendication 7, **caractérisé en ce que** la vitesse de rotation des récipients à partir du premier segment de chemin (A) est augmentée avec une accélération définie jusqu'à une vitesse de rotation maximale V_{Rot_max}, et **en ce que** l'accélération est commandée en tenant compte de la vitesse de transport V_{Trans} du premier dispositif de transport (3).

13. Procédé selon au moins une des revendications 7 et 8, **caractérisé en ce que** la vitesse de rotation entre le premier segment de chemin (A) et le deuxième segment de chemin (C) est maintenue sensiblement constante.
